# EUROPEAN PATENT SPECIFICATION

(11) **EP 4 615 399 B1**
(45) Date of publication and mention of the grant of the patent: **20.05.2026**
(21) Application number: 23793728.9
(22) Date of filing: 18.10.2023
(51) Int. Cl.: A61K 8/34, A61K 8/37, A61K 8/81, A61K 8/92, A61K 8/9789, A61Q 19/02, A61K 9/00, A61K 9/06, A61K 47/44

(54) **A SKIN BRIGHTENING COMPOSITION**
HAUTAUFHELLUNGSZUSAMMENSETZUNG
COMPOSITION D'ÉCLAIRCISSEMENT DE LA PEAU

(30) Priority: 08.11.2022 EP 22205973
(43) Date of publication of application: 17.09.2025
(73) Proprietor: Unilever IP Holdings B.V., 6708 WH Wageningen (NL); Unilever Global IP Limited, Wirral, Merseyside CH62 4ZD (GB)
(72) Inventor: BETADPUR, Anagha, 6708 WH Wageningen (NL); DASGUPTA, Anindya, 6708 WH Wageningen (NL); DUTTA, Maitreyee, 6708 WH Wageningen (NL); KUMARAN, Srikala, 6708 WH Wageningen (NL); NAIR, Nirmala Santosh, 6708 WH Wageningen (NL)
(74) Representative: Unilever Patent Group
(86) International application number: PCT/EP2023/078933
(87) International publication number: WO 2024/099715

(56) References cited:
- US-A1- 2012 128 605
- US-A1- 2017 128 357
- DR CI:LABO: "BB Perfect Cream Foundation SPF 35 PA+++", GNPD, MINTEL, 7 February 2018 (2018-02-07), XP002777945

## Description

### Field of the Invention

The present invention relates to a personal care composition which delivers bright and even skin tone.

### Background of the Invention

Most people consider skin appearance especially on the face as one of the key indicators of their own beauty and health. Having an even skin tone which is bright and free of blemishes is thus desired by many. The degree and evenness of pigmentation of the skin is affected by factors like age, hormonal changes, occurrence of acne and exposure to sunlight and pollution. These can cause spots or freckles, hyper-pigmentation on certain localised areas of skin and also under-eye dark circles. Many people believe that certain life-style factors like hydration (quantity of water consumed), the type of food and quantity and quality of sleep also have an effect on skin appearance including the dark circles under the eyes. Since people are aware that changes like leading a healthier lifestyle may take a long time for evening out skin appearance, they rely on cosmetic solution to give them temporary solution to blemishes on their skin. They also seek such products to reduce the skin darkening caused by exposure to sunlight. To meet this need, many attempts have been made to develop products that reduce the pigment production in the melanocytes.

The present inventors have been working in this area for a long time and have also filed several patent applications and produced various cosmetic products which are in the market. In the present invention, they were specifically looking for a highly effective solution to this problem which could also be used as a general cosmetic product for delivering even and bright skin tone especially on exposed skin surfaces.

During the course of their extensive research in understanding the factors and actives that affect pigmentation and their exploration into area of human skin microbiome which are two apparently unrelated areas, they found to their surprise that a combination of a well known skin brightening active, a resorcinol derivative, in combination with Moringa seed oil (that they found is a suitable prebiotic for skin microbiome) that is fermented by skin commensal bacteria, interact synergistically to deliver even and bright skin tone. Oil from moringa has been used safely on skin for a long time and therefore provides a natural alternative to chemical actives which are considered harsh by some people. To the knowledge of the present inventors such a combination of actives to deliver this benefit is not known before.

It is thus an object of the present invention to provide for a solution to the problem of uneven skin appearance by delivering a topical composition that gives even and brighter skin appearance.

It is another object of the present invention to provide such a solution which involves inclusion of actives which are mild and known to be safe on skin.

### Summary of the Invention

The first aspect of the present invention relates to a personal care composition comprising:
(a) moringa seed oil;
(b) a substituted resorcinol; and
(c) a cosmetically acceptable carrier,
wherein the moringa seed oil is fermented by skin commensal bacteria.

Another aspect of the present invention relates to a non-therapeutic method of providing brightness to skin, comprising the step of applying a composition of the first aspect on to skin.

### Detailed Description of the Invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated; and may be abbreviated as 'wt%'. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The composition of the invention is meant to be used for personal care or for cosmetic use and could also be referred to as a personal care composition or a cosmetic composition. By a "personal care composition" as used herein, is meant to include a composition for topical application i.e external surfaces of the skin of humans. Such a composition may be classified as leave-on or rinse off, and includes any product applied to a human body for improving appearance, cleansing, odour control or general aesthetics. The composition is preferably of the leave-on type. The composition of the present invention can be in the form of a liquid, lotion, cream, foam, stick, serum, essence or gel. Preferred compositions include leave-on gels, lotions, serum or creams, preferably it is in the serum or cream form. "Skin" as used herein is meant to include skin on the face and body (e.g., neck, chest, back, arms, underarms, hands, legs and scalp) and especially to the exposed parts thereof.

The present invention provides for a personal care composition comprising moringa seed oil; a substituted resorcinol; and a cosmetically acceptable carrier, wherein the moringa seed oil is fermented by skin commensal bacteria (the composition).

The genus Moringa comprises about fourteen species of plants (of which particularly *Moringa peregrina, M. aptera, M. concanensis, M. drouhardii, M. hildebrandtii, M. longituba), among which Moringa pterygosperma* are the best known. The seeds of Moringa are characterized by the presence of an oil whose content could vary from 20 to 80% according to the species and maturity of the seeds. For the species *Moringa oleifera*, the oil content mentioned in the literature ranges from 21 to 34%.

The comparison of the oils of the seeds of *Moringa oleifera, M. peregrina, M. concanensis* and *M. drouhardii* show a very similar fatty acid content, these oils all having predominantly oleic acid along with some saturated fatty acids. It is preferred that the oil is extracted from seeds of at least one of *Moringa oleifera, Moringa pterygosperma, Moringa peregrina, Moringa concanensis* or *Moringa drouhardii*. It is particularly preferred that the oil is extracted from *Moringa oleifera or Moringa pterygosperma*. Preferably the amount of the oil in the compositions of the invention is 0.015 to 0.4 wt%. The oil is rich in oleic fatty acids. It is particularly preferred that the moringa seed oil for use in the present invention comprises 60 to 80% oleic acid and 2-10% palmitic acid.

In the Int. J. Mol. Sci. 2016, 17, 2141; doi:10.3390/ijms17122141, Leone et.al., have disclosed a review of literature concerning the composition of Moringa oil.

Preferably the oil is extracted almost entirely by solvent extraction, particularly using n-hexane, more preferably at extraction ratio of from 3:1 to 8:1 parts by weight. Alternatively, the oil is extracted by cold press extraction. It is reported that about 69% (on average) of the total oil contained in seeds can be extracted by cold press.

The composition comprises the moringa seed oil that is fermented by skin commensal bacteria. Preferably, such commensal bacteria include one or more of *Staphylococcus epidermidis, Staphylococcus hominis, Cutibacterium acnes*, *Micrococcus luteus*, *Corynebacterium* species e.g. *Corynebacterium xerosis*, *Corynebacterium pseudogenitalium* and *Corynebacteium tuberculostearicum*. The moringa seed oil is preferably included in 0.01 to 1%, preferably 0.05 to 1%, preferably from 0.1 to 1%, preferably 0.5 to 1% by weight of the composition. It is expected that the benefits of the invention could be obtained even with application of (unfermented) moringa seed oil in the composition of the invention, on the skin. The moringa seed oil when so applied is expected to interact with commensal bacteria described above, e.g. *S. epidermidis,* normally present on the skin, to ferment the moringa seed oil *in-situ* thereon, to deliver the same benefits as obtained when the moringa seed oil is fermented *ex-situ*. Thus, moringa seed oil used in the composition can be fermented either *ex-situ* or *in-situ* by skin commensal bacteria.

Resorcinol is a dihydroxy phenol compound (i.e., 1,3-dihydroxybenzene) characterized by alcohol groups (-OH) at positions 1 and 3. The chemical structure of resorcinols may be modified, resulting in substituted resorcinols characterized by at least one substituent in the 2, 4, 5 or 6 position. It is preferred that the resorcinol comprises at least one substituent comprising 2 to 11 carbon atoms, preferably, 2 to 8 carbon atoms, most preferably 2 to 6 carbon atoms. It is particularly preferred that at least one substituent comprises an alkyl group. The resorcinol compounds comprised in the cosmetic compositions of the present invention are oil-soluble substituted resorcinols.

Preferably, the resorcinol of the cosmetic composition is only substituted at position 4 preferably with an alkyl group. Illustrated but not limiting examples of substituted resorcinols include 4-ethyl resorcinol, 4-hexylresorcinol, 4-phenylethylresorcinol, 4-cyclopentyl resorcinol, 4-cyclohexylresorcinol, 4-octylresorcinol, and mixtures thereof. The more preferred substituted resorcinol for use in the present invention is one or both of 4-ethyl resorcinol and 4-hexyl resorcinol; most preferred being 4-hexyl resorcinol. The composition preferably comprises 0.00001 to 1%, preferably, 0.0001 to 1%, preferably 0.001 to 1%, preferably 0.01 to 1% and preferably 0.1 to 0.5% e.g. 0.25% substituted resorcinol by weight of the cosmetic composition, including all ranges subsumed therein.

A benefit of the present invention is that it has been found that with the inclusion of fermented moringa seed oil which is a natural material, preferably much lower amount of a synthetic active like substituted resorcinol needs to be included to get the same efficacy.

Without wishing to be bound by theory the inventors believe that the combination of the actives work to deliver the benefits of the present invention by way of inhibiting tyrosinase enzyme activity and its levels in melanocytes.

The composition preferably comprises an oil which has a Hansen total solubility parameter (δₜ) value within the range of 16.5 to 22. The preferred oils may be selected from one or more of isopropyl myristate, isopropyl palmitate, caprylic/capric triglycerides and benzyl alcohol. These oils are preferably included in an amount of 8 to 25% by weight of the composition.

An emulsifying polymer is preferably included in the composition of the invention. The emulsifying polymer is preferably a swellable polymer powder made of pure acrylic acid monomers or a mixture of acrylic acid and methacrylic acid monomers, and are, preferably, polymers having a chemical name of Acrylates/C₁₀-₃₀ Alkyl Acrylate Crosspolymer, Carbomer, or a mixture thereof. In one aspect, the polymer is a crosslinked or non-crosslinked homopolymer. In another aspect, the polymer is a crosslinked or non-crosslinked copolymer. The emulsifying polymer is multifunctional and is capable of emulsifying, stabilizing, and/or building viscosity of a composition. It is within the scope of the present compositions to employ a polymeric emulsifier alone or in combination with other additional polymeric emulsifiers. Illustrative but nonlimiting Acrylates/C₁₀-₃₀ Alkyl Acrylate Crosspolymer for use in the present cosmetic compositions are made commercially available by the supplier The Lubrizol Corporation under such trade names as PEMULEN^{™} TR-1, PEMULEN^{™} TR-2, PEMULEN^{™} EZ-4U, CARBOPOL^{®} Ultrez 20, CARBOPOL^{®} Ultrez 21, CARBOPOL^{®} 1382 and CARBOPOL^{®} ETD 2020. Desirable Carbomers for use in the present composition include CARBOPOL^{®} Ultrez 10 and CARBOPOL^{®} 980, both commercially sold by The Lubrizol Corporation. Emulsifying polymers are included in the cosmetic composition in an amount of 0.01 to 5%, preferably, from 0.02 to 4%, and, more preferably, from 0.03 to 3%, by total weight of the composition. It is still more preferred that the polymers are included in the cosmetic composition at 0.04 to 2% by weight, most preferably, 0.05 to 1% by weight of the cosmetic composition, including all ranges subsumed therein.

The composition of the invention may additionally comprise one or more of a sunscreen, photostabilizer, skin-brightening agent, wrinkle-reducing agent, and coloring agent.

The composition may additionally comprise an organic sunscreen selected from one or both of a UVA sunscreen and a UVB sunscreen. Sunscreens include those materials commonly employed to block ultraviolet light. Illustrative compounds are the derivatives of PABA, cinnamate and salicylate. For example, avobenzophenone (Parsol 1789^{®}) octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively. The exact amount of sunscreen employed in the compositions can vary depending upon the degree of protection desired from the sun's UV radiation. Additives that reflect or scatter the sun rays may also be employed. These additives include oxides like zinc oxide and titanium dioxide.

The composition of the present invention may further comprise a cosmetically acceptable carrier, which may act as diluents, dispersants. and/or carriers for the actives used in the composition, so as to facilitate their distribution when the composition is applied to the skin. The cosmetically acceptable vehicle suitable for use in the present invention may be aqueous, anhydrous or an emulsion; preferably aqueous or an emulsion, with the emulsion being a water-in-oil or oil-in-water emulsion, the latter being most preferred. Water when present typically makes up the balance of the composition. Preferably water is present in a concentration of 5 to 99%, more preferably from 20 to 80%, still more preferably from 40 and 80% by weight of the composition.

The composition of the present invention may be delivered in a serum, cream, lotion or gel form preferably in cream form. A preferred format for the solid form of the composition is a cream, further more preferably one which has a vanishing cream base. Vanishing cream base is one which comprises 3 to 25 wt% fatty acid. Optionally, the composition may comprise 0.1 to 10 wt% soap. When included, the fatty acid is preferably a C10 to C22 fatty acid, more preferably a C16 to C18 fatty acid. Most preferably the fatty acids are stearic acid or palmitic acid or a mixture thereof and the soap is preferably the potassium salt of the fatty acid mixture. The fatty acid is often hystric acid which is substantially (generally about 90 to 95 %) a mixture of 45 % stearic acid and 55 % palmitic acid. The most preferred cream is one having 3 to 25 wt% fatty acid and 0.1 to 10 wt% soap.

Preferably, the composition comprises emollients. Examples of emollients that may be used in the leave-on composition include stearyl alcohol, glyceryl monoricinoleate, mink oil, isopropyl isostearate, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, din-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isopropyl linoleate, lauryl lactate, myristyl lactate, decyl oleate, myristyl myristate and mixtures thereof.

Preferably, the composition comprises solvents. Examples of solvents that may be used in the composition include ethyl alcohol, isopropanol, acetone, ethylene glycol mono ethyl ether, diethylene glycol mono butyl ether, diethylene glycol mono ethyl ether and mixtures thereof. The composition may comprise polyhydric alcohols which may be selected from one or more of glycerine, 1,3-butylene glycol, propylene glycol, 1,3-propanediol, pentylene glycol, hexylene glycol, and sorbitol.

Preferably, the composition comprises powders. Examples of powders that may be used in the composition include chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate and mixtures thereof.

Preferably, the composition comprises preservatives to protect against the growth of potentially harmful microorganisms. Examples of ingredients that may be used as preservatives in the composition include alkyl esters of para-hydroxybenzoic acid, hydantoin derivatives, propionate salts, and a variety of quaternary ammonium compounds. More preferably, ingredients that may be used as preservative in the composition are sodium benzoate, iodopropynyl butyl carbamate, methylisothiazolinone, iodopropynylbutylcarbamate, phenoxyethanol, methyl paraben, propyl paraben, imidazolidinyl urea, sodium dehydroacetate, ethylhexylglycerin, benzyl alcohol, alkane diols and mixtures thereof. When present in the composition, preservatives are added preferably in an amount 0.001 to 5 wt%, more preferably 0.01 to 3 wt% and most preferably 0.02 to 2 wt%, even most preferably 0.25 to 1.5%.

Preferably, the composition comprises a range of other optional ingredients that include antioxidants, binders, buffering agents, colorants, astringents, fragrance, opacifying agents, conditioners, exfoliating agents, pH adjusters, skin sensates, skin soothing agents, and skin healing agents.

It is within the scope of the present invention that the compositions claimed here are ecofriendly and therefore are free of certain ingredients that are increasingly being implicated in long term detriment to the environment. Thus, one aspect of the present invention relates to a composition which preferably is free of sulphate surfactants. Another aspect relates to compositions herein that are preferably free of preservatives. Yet another aspect relates to compositions that are preferably free of acrylate polymers. Also within the scope of the present invention are composition which are preferably free of titanium dioxide. Green (i.e. biodegradable chelating agents are preferred) for use in such compositions i.e the composition is preferably free of conventional chelating agents like EDTA. By "free of" a certain ingredient, as per this present invention, it is preferably meant that the composition comprises less than 0.1, preferably less than 0.05, further more preferably less than 0.01% of the ingredient by weight of the composition. They are optimally absent from the composition.

The packaging for the composition of this invention can be a patch, a bottle, a tube, a roll-ball applicator, a propellant driven aerosol device, a squeeze container or a lidded jar.

In an alternate aspect, the invention relates to a non-therapeutic use of the composition according to the invention for skin brightening. The use is non-therapeutic or cosmetic in nature.

In yet another aspect, the invention relates to a non-therapeutic method of providing brightness or even tone to skin of a human, the method comprising the step of applying the composition according to the invention onto the skin. The method is non-therapeutic or cosmetic in nature.

In yet another aspect of the present invention, there is provided a non-therapeutic method of providing microbiome benefit to skin comprising the step of applying a composition according to the present invention on to skin. The method is non-therapeutic or cosmetic in nature.

The invention will now be illustrated by means of the following non-limiting examples.

### Examples

### Examples A to K, 1 to 4: % melanin reduction achieved by the combination of actives as per the invention vs. Control

Various samples containing actives were prepared and the % melanin reduction was measured. The protocol for measuring the melanin reduction is summarized below:
Co-cultures of HaCat keratinocytes and primary human melanocytes were mixed at 40,000 each (1:1 ratio) in 1 ml of a 1:1 mix of their respective culture medium and seeded as such per well, in 12 well plates. After 24 hrs, cultures were treated with various concentrations of the test materials shown in table 1 and left undisturbed for a further period of 72 hrs. Comparative vehicle control was also set up simultaneously. For melanin estimation at the end of the 72 hrs incubation, cell viability was first determined using the Calcein AM method. Briefly, spent media was removed and cells were washed once with 0.4ml of 1x PBS/well. Fresh 1µM calcein-AM in PBS buffer was added to each well, including control wells without cells. Plates were covered with aluminium foil and incubated for 30mins at 37°C in a CO₂ incubator. Calcein fluorescence was measured (excitation at 490 nm and emission at 520 nm) in a TECAN M1000 plate reader. Culture wells were drained and lysed using 1N NaOH containing 10% DMSO and placed for 1 hour at 60°C in a shaker incubator. 100µl of the supernatant was transferred to a 384 well plate and melanin was measured in a TECAN plate reader (405nm filter). Melanin content (MC) was expressed after correction for cell numbers (MC/Calcein) and represented as %melanin.

The following samples as given in Table -1 were prepared and the % melanin reduction of each as measured, are also given in the table.

**Table - 1**

| Example | Actives used (wt%) | % melanin reduction |
|---|---|---|
| A | DMSO Control | 0 |
| B | 0.0002% 4-HR | 23 |
| C | 0.00002% 4-HR | 3 |
| D | 0.05% moringa oil fermented (1:40) | 19 |
| E | 0.01% moringa oil fermented (1:40) | 15 |
| F | 0.05% moringa oil (un-fermented) | -1 |
| G | 0.01% moringa oil (un-fermented) | -3 |
| H | 4-HR 0.0002%+Moringa oil 0.05% (un-fermented) | 9 |
| I | 4-HR 0.0002%+Moringa oil 0.01% (un-fermented) | 18 |
| J | 4-HR 0.00002%+Moringa oil 0.05% (un-fermented) | 0 |
| K | 4-HR 0.00002%+Moringa oil 0.01% (un-fermented) | 1 |
| 1 | 0.0002% 4-HR + 0.05% moringa oil fermented (1:40) | 31 |
| 2 | 0.00002% 4-HR + 0.05% moringa oil fermented (1:40) | 23 |
| 3 | 0.00002% 4-HR + 0.01% moringa oil fermented (1:40) | 24 |
| 4 | 4-HR 0.0002%+Moringa oil fermented 0.01% 1:40 | 21 |

In the above table:
Moringa seed oil was sourced from Naturex SA, France.
Moringa oil was fermented as mentioned below.

Tryptic Soya agar (TSA) was prepared and autoclaved. TSA plates were poured and allowed to solidify followed by streaking of *S*. *epidermidis* (ATCC 12228) from the glycerol stocks on it and incubated for 24 hours. Moringa oil was dissolved in DMSO and then added to autoclaved Tryptic Soya Both (TSB) at required concentration of 0.05% and 0.01%. 0.3 O.D. of pure bacterial culture was made and inoculated in autoclaved TSB with the Moringa oil and incubated for 24 hours in shaker incubator at 120 rpm at 37° C (in a sample cup). After 24 hours incubation, O.D. was measured to check there is no interference of the bio-active in the growth of the bacteria. The culture was transferred to a centrifuge tube and centrifuged at 6500 rpm for 10 minutes. The supernatant was filter sterilized using a membrane filter, aliquoted up to 1 ml and stored at -80°C for testing on skin cell cultures.

The *S*. *epidermidis* fermented Moringa oil samples were diluted 1:40 in the keratinocyte+melanocyte media mix before being introduced to the co-cultures for melanin content assay.

The data in the table -1 above indicates that composition as per the invention (Example 1) provides significantly superior reduction in melanin content as compared to use of 4-HR at 0.0002% concentration (Example - B). It has been found that use of 4-HR at concentrations higher than 0.0002% (at the cellular level in invitro experiments) leads to cell viability problems. Further Example - 3 demonstrates synergistic interaction between 4-HR and moringa seed oil.

It is to be understood that the experiments described above were conducted in an in-vitro assay.

It is expected that the concentrations to be actually used to prepare a composition for topical use would be vastly different. The concentrations could be orders of magnitude higher due to the following reasons that affect the difference in concentration in the bulk as compared to that at the cellular level. The composition may be formulated as an emulsion or a gel with very many additional ingredients. The concentration of the desired actives in the oil phase and in the water phase, is expected to be very different. They may also have very different physical and hydrodynamic properties like partition coefficients, diffusional rates, convective transport rates, rheological properties etc. Therefore, it is expected that the concentrations to be used when formulated as a composition would be very different from that evaluated at the cellular level. The use when formulated as a composition is usually very much higher even two to three orders of magnitude higher, than that at which the invitro experiments are carried out at the cellular levels.

## Claims

1. A personal care composition comprising:
(a) moringa seed oil;
(b) a substituted resorcinol; and
(c) a cosmetically acceptable carrier,
wherein the moringa seed oil is fermented by skin commensal bacteria.

2. A composition as claimed in claim 1, wherein the substituted resorcinol comprises one or more of 4-hexylresorcinol, 4-ethylresorcinol, 4-phenylethylresorcinol, 4-cyclopentylresorcinol, 4-cyclohexylresorcinol, and 4-octylresorcinol.

3. A composition as claimed in claim 2 wherein the substituted resorcinol comprises one or both of 4-hexylresorcinol and 4-ethylresorcinol, preferably 4-hexyl resorcinol.

4. A composition as claimed in any one of the preceding claims comprising 0.00001 to 1% by weight of substituted resorcinol.

5. A composition as claimed in any one of the preceding claims wherein the moringa seed oil comprises 60 to 80% oleic acid and 2-10% palmitic acid.

6. A composition as claimed in any one of the preceding claims wherein the skin commensal bacteria are one or more of *Staphylococcus epidermidis, Staphylococcus hominis, Cutibacterium acnes, Micrococcus luteus, Corynebacterium species.*

7. A composition as claimed in any one of the preceding claims comprising 0.01 to 1% by weight moringa seed oil.

8. A composition as claimed in any one of the preceding claims wherein the cosmetically acceptable carrier comprises an oil preferably selected from one or more of isopropyl myristate, isopropyl palmitate, caprylic/capric triglycerides and benzyl alcohol.

9. A composition as claimed in any one of the preceding claims wherein the cosmetically acceptable carrier comprises an emulsifying polymer selected from one or more of an acrylates/C₁₀-₃₀ alkyl acrylate crosspolymer, and carbomer preferably included in 0.01 to 5% by weight of the composition.

10. A composition as claimed in any one of the preceding claims in serum, cream, lotion or gel form preferably in serum or cream form.

11. A non-therapeutic method of providing even tone to skin comprising the step of applying a composition as claimed in any one of the preceding claims on to skin.

## Patentansprüche

1. Körperpflegezusammensetzung, die umfasst:
(a) Moringa-Samenöl;
(b) ein substituiertes Resorcin; und
(c) einen kosmetisch verträglichen Träger,
wobei das Moringa-Samenöl durch hautkommensale Bakterien fermentiert ist.

2. Zusammensetzung, wie im Anspruch 1 beansprucht, wobei das substituierte Resorcin eines oder mehrere von Folgenden umfasst: 4-Hexylresorcin, 4-Ethylresorcin, 4-Phenylethylresorcin, 4-Cyclopentylresorcin, 4-Cyclohexylresorcin und 4-Octylresorcin.

3. Zusammensetzung, wie im Anspruch 2 beansprucht, wobei das substituierte Resorcin 4-Hexylresorcin und/oder 4-Ethylresorcin, vorzugsweise 4-Hexylresorcin, umfasst.

4. Zusammensetzung, wie in irgendeinem der vorhergehenden Ansprüche beansprucht, die 0,00001 bis 1 Gew.-% substituiertes Resorcin umfasst.

5. Zusammensetzung, wie in irgendeinem der vorhergehenden Ansprüche beansprucht, wobei das Moringa-Samenöl 60 bis 80% Ölsäure und 2 bis 10% Palmitinsäure umfasst.

6. Zusammensetzung, wie in irgendeinem der vorhergehenden Ansprüche beansprucht, wobei die hautkommensalen Bakterien eines oder mehrere von Folgenden sind: Staphylococcus epidermidis, Staphylococcus hominis, Cutibacterium acnes, Micrococcus luteus, Corynebacterium-Arten.

7. Zusammensetzung, wie in irgendeinem der vorhergehenden Ansprüche beansprucht, die 0,01 bis 1 Gew.-% Moringa-Samenöl umfasst.

8. Zusammensetzung, wie in irgendeinem der vorhergehenden Ansprüche beansprucht, wobei der kosmetisch verträgliche Träger ein Öl umfasst, das vorzugsweise unter einem oder mehreren von folgenden ausgewählt ist: Isopropylmyristat, Isopropylpalmitat, Capryl-/Capric-Triglyceriden und Benzylalkohol.

9. Zusammensetzung, wie in irgendeinem der vorhergehenden Ansprüche beansprucht, wobei der kosmetisch verträgliche Träger ein emulgierendes Polymer umfasst, das unter einem oder mehreren von folgenden ausgewählt ist: einem Acrylate/C₁₀₋₃₀-Alkylacrylat-Kreuzpolymer und Carbomer, wobei das Carbomer vorzugsweise in einer Menge von 0,01 bis 5 Gew.-% der Zusammensetzung enthalten ist.

10. Zusammensetzung, wie in irgendeinem der vorhergehenden Ansprüche beansprucht, in Form eines Serums, einer Creme, einer Lotion oder eines Gels, vorzugsweise in Form eines Serums oder einer Creme.

11. Nicht-therapeutisches Verfahren zum Erzeugen eines gleichmäßigen Hauttons, umfassend den Schritt des Aufbringens einer Zusammensetzung, wie in irgendeinem der vorhergehenden Ansprüche beansprucht, auf die Haut.

## Revendications

1. Composition de soin personnel comprenant :
(a) de l'huile de graines de moringa ;
(b) un résorcinol substitué ; et
(c) un véhicule acceptable en cosmétique,
dans laquelle l'huile de graines de moringa est fermentée par des bactéries commensales de la peau.

2. Composition selon la revendication 1, dans laquelle le résorcinol substitué comprend un ou plusieurs parmi le 4-hexylrésorcinol, le 4-éthylrésorcinol, le 4-phényléthylrésorcinol, le 4-cyclopentylrésorcinol, le 4-cyclohexylrésorcinol, et le 4-octylrésorcinol.

3. Composition selon la revendication 2, dans laquelle le résorcinol substitué comprend l'un ou les deux parmi le 4-hexylrésorcinol et le 4-éthylrésorcinol, de préférence le 4-hexylrésorcinol.

4. Composition selon l'une quelconque des revendications précédentes, comprenant 0,00001 à 1 % en poids de résorcinol substitué.

5. Composition selon l'une quelconque des revendications précédentes, dans laquelle l'huile de graines de moringa comprend 60 à 80 % d'acide oléique et 2 à 10 % d'acide palmitique.

6. Composition selon l'une quelconque des revendications précédentes, dans laquelle les bactéries commensales de la peau sont une ou plusieurs parmi *Staphylococcus epidermidis, Staphylococcus hominis, Cutibacterium acnes, Micrococcus luteus,* et les espèces de *Corynebacterium.*

7. Composition selon l'une quelconque des revendications précédentes, comprenant 0,01 à 1 % en poids d'huile de graines de moringa.

8. Composition selon l'une quelconque des revendications précédentes, dans laquelle le véhicule acceptable en cosmétique comprend une huile qui est de préférence un ou plusieurs choisis parmi le myristate d'isopropyle, le palmitate d'isopropyle, les triglycérides capryliques/ capriques et l'alcool benzylique.

9. Composition selon l'une quelconque des revendications précédentes, dans laquelle le véhicule acceptable en cosmétique comprend un polymère émulsifiant qui est un ou plusieurs choisis parmi un polymère réticulé d'acrylates/acrylate d'alkyle en C₁₀ à C₃₀, et un carbomère, de préférence présent à raison de 0,01 à 5 % en poids de la composition.

10. Composition selon l'une quelconque des revendications précédentes, sous la forme d'un sérum, d'une crème, d'une lotion ou d'un gel, de préférence sous la forme d'un sérum ou d'une crème.

11. Méthode non thérapeutique pour conférer un teint uniforme à la peau, comprenant l'étape d'application sur la peau d'une composition selon l'une quelconque des revendications précédentes.
